# EUROPEAN PATENT APPLICATION

(11) **EP 4 567 723 A1**
(43) Date of publication of application: **11.06.2025**
(21) Application number: 23850153.0
(22) Date of filing: 03.08.2023
(51) Int. Cl.: G06T 7/00, G06V 10/70, A61B 5/00, A61B 5/107

(54) **SKIN CONDITION ESTIMATING METHOD**

(30) Priority: 04.08.2022 JP 2022124831; 29.03.2023 JP 2023052673
(71) Applicant: KAO CORPORATION, Tokyo 103-8210 (JP)
(72) Inventor: NISHINO, Ken, Odawara-shi, Kanagawa 250-0002 (JP)
(74) Representative: Hoffmann Eitle
(86) International application number: PCT/JP2023/028461
(87) International publication number: WO 2024/029600

(57) **Abstract**

In a skin condition estimating method, one or more processors, capable of using a learned model for estimating a skin condition of a human face appearing in an input face image from the input face image having a predetermined rectangular shape with a predetermined image size, execute a process of acquiring a face image of a subject, a process of identifying position coordinates of a plurality of points in a face region of a human face appearing in the acquired face image, a process of converting the face image by coordinate-converting some or all of the position coordinates of the identified plurality of points so that the face region has a predetermined image size and a predetermined rectangular shape, and a process of inputting the converted face image to the learned model to acquire skin condition information of the face of the subject.

## Description

### TECHNICAL FIELD

The present invention relates to a technique of estimating a skin condition of a human face appearing in an input face image from the input face image using a learned estimating model.

### BACKGROUND ART

Patent Document 1 below discloses a system for determining an apparent skin age of a person by masking a macroscopic feature of a face in an image of the person and then analyzing the image using a convolutional neural network, and displaying the determined apparent skin age on a display device visually recognizable to a user.

### CITATION LIST

Patent Document 1: WO 2018/222808 A

### SUMMARY OF THE INVENTION

According to the present invention, there is provided a skin condition estimating method using an estimating model learned to output a skin condition of a human face appearing in an input face image from the input face image, the method in which one or more processors execute: an image acquisition process of acquiring a face image of a subject; a conversion process of converting the face image so that a plurality of points on a contour of the face region of the human face appearing in the acquired face image is positioned at an outer edge of an image in order to match an input rule in the estimating model to obtain a converted face image; and an information acquisition process of acquiring skin condition information of the face of the subject output from the estimating model by inputting the converted face image as the input face image.

Furthermore, according to the present invention, there is provided a skin condition estimating system including: an estimating model learned to output a skin condition of a human face appearing in an input face image from the input face image; an image acquisition unit configured to acquire a face image of a subject; a conversion unit configured to obtain a converted face image by converting the face image so that a plurality of points on a contour of a face region of the human face appearing in the acquired face image is positioned at an outer edge of an image in order to match an input rule in the estimating model; an information acquisition unit configured to input the converted face image to the estimating model as the input face image and acquire skin condition information of the face of the subject output from the estimation model; a skin condition output unit configured to generate image information for presenting the acquired skin condition information; and an information display unit configured to display the image information.

Furthermore, according to the present invention, it is possible to provide a user terminal for the skin condition estimating system described above.

In addition, it is also possible to provide a program for causing a computer to execute the above skin condition estimating method, that is, causing the computer to function as all or a part of the above skin condition estimating system, and it is also possible to provide a recording medium readable by a computer recording such a program. The recording medium includes a non-transitory tangible medium.

### BRIEF DESCRIPTION OF THE DRAWINGS

Fig. 1 is a diagram conceptually illustrating an example of a hardware configuration of an information processing apparatus capable of executing a skin condition estimating method according to the present embodiment.
Fig. 2 is a block diagram for explaining functions of respective units of a skin condition estimating apparatus according to the present embodiment.
Fig. 3 is a flowchart illustrating the skin condition estimating method according to the present embodiment.
Fig. 4 is a diagram illustrating a flow of conversion of a face image in the skin condition estimating method according to the present embodiment.
Fig. 5 is a flowchart illustrating a learning method of a skin condition estimating model according to the present embodiment.
Fig. 6 is a diagram illustrating a flow of conversion of a face image in a skin condition estimating method according to a modification.
Fig. 7 is a flowchart illustrating the skin condition estimating method according to the modification.
Fig. 8 is a diagram functionally illustrating an embodiment in which a skin condition estimating system is configured as a combination of a user terminal and a server.
Fig. 9 is a diagram functionally illustrating another embodiment in which the skin condition estimating system is configured as a combination of the user terminal and the server.
Fig. 10 is a diagram illustrating an example of a display screen of skin condition information on the user terminal.
Fig. 11 is a diagram illustrating a flow of conversion of a face image in a skin condition estimating method according to an example.
Fig. 12 is a graph illustrating a difference in prediction error between the skin condition estimating method (the present method) according to the present embodiment, the conventional method, and the prior patent method.
Fig. 13 is a diagram illustrating an example of face image conversion using a coordinate conversion rule in which the enlargement ratio around an outer corner of an eye is made larger than that of other parts in order to estimate a wrinkle at the outer corner of the eye in more detail.
Fig. 14 is a diagram illustrating an example of face image conversion using the coordinate conversion rule for making the enlargement ratio around a nasolabial fold larger than that of other parts in order to estimate a state of the nasolabial fold in more detail.

### DESCRIPTION OF EMBODIMENTS

In the existing method of estimating the skin condition of the human face appearing in the face image using the learned estimating model like the system described as the background art, the face image input to the estimating model is normalized to a predetermined image size and a predetermined rectangular shape, and the input face image is analyzed. On the other hand, the face image input to the estimating model includes a region other than the face, such as a background and hair, in addition to a face region.

Therefore, the estimating model also needs to learn not to use a non-skin-like region in addition to the skin condition of the face region in the learning process and needs to hold the knowledge resource learned in such a manner.

However, it can be said that calculation and information regarding a region other than the face are useless for the estimating model to estimate the skin condition of the human face. In addition, when the image size is compressed in a state where a region other than the face is included in the normalization of the face image, the resolution of the face region decreases, and thus, the estimation accuracy of a learned model may also decrease.

The present invention provides a technique for improving estimation accuracy of an estimating model for estimating a skin condition of a human face appearing in an input face image from the input face image in consideration of the above-described viewpoint.

According to the present invention, it is possible to provide a technique for improving estimation accuracy of an estimating model that estimates a skin condition of a human face appearing in an input face image from the input face image.

Hereinafter, examples of preferred embodiments of the present invention (hereinafter, referred to as the present embodiment) will be described. Note that the present embodiment described below is an example, and the present invention is not limited to the following configuration.

### [Hardware Configuration Example]

The skin condition estimating method (hereinafter, it is referred to as the present method) according to the present embodiment is executed by one or more processors included in one or more information processing apparatuses.

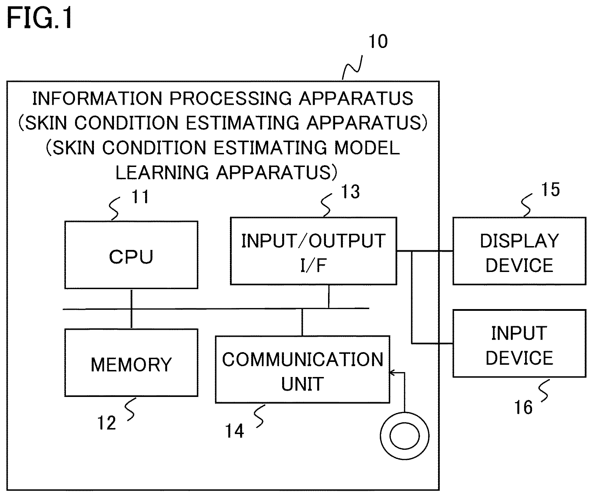

Fig. 1 is a diagram conceptually illustrating a hardware configuration example of an information processing apparatus 10 capable of executing the present method.

The information processing apparatus 10 is a so-called computer, and includes a CPU 11, a memory 12, an input/output interface (I/F) 13, a communication unit 14, and the like. The information processing apparatus 10 may be a stationary personal computer (PC) or a portable terminal such as a portable PC, a smartphone, a tablet, and the like.

The CPU 11 is a so-called microprocessor. The memory 12 is a random access memory (RAM), a read only memory (ROM), or an auxiliary storage device (such as a hard disk).

The input/output I/F13 can be connected to a user interface device such as a display device 15 and an input device 16. The display device 15 is a device that displays a screen corresponding to drawing data prepared by the CPU 11 or the like, such as a liquid crystal display (LCD). The input device 16 is a device that receives an input of a user operation such as a keyboard and a mouse. The display device 15 and the input device 16 may be integrated and realized as a touch panel.

The communication unit 14 communicates with other computers via a communication network, exchanges signals with other devices such as a printer, and the like. A portable recording medium or the like can also be connected to the communication unit 14.

The hardware configuration of the information processing apparatus 10 is not limited to the example of Fig. 1. The information processing apparatus 10 may include other hardware elements not shown in the drawings. In addition, the number of hardware elements is not limited to the example of Fig. 1. For example, the information processing apparatus 10 may include a plurality of CPUs 11. In particular, when a graphics processing unit (GPU) designed for vector operation is used instead of a general CPU in order to realize a convolutional neural network that estimates the skin condition, the operation speed can be increased.

Furthermore, the present invention may be realized as a so-called system by a plurality of computers formed by combining a plurality of information processing apparatuses 10. In a case where the present invention is realized by the plurality of computers, as will be described later, it is preferable that the plurality of computers be made to share each function and be operated as a system that functions by combining a server and a terminal connected by communication via a network such as the Internet. Note that, in the following description, a form in which the present invention is implemented by the information processing apparatus 10 will be described as an example.

The information processing apparatus 10 executes the present method by the CPU 11 executing a computer program stored in the memory 12. This computer program is temporarily installed from, for example, a portable recording medium such as a compact disc (CD), a memory card, and the like, or another computer on a network via the input/output I/F13 or the communication unit 14, and then directly stored and executed in the memory 12, whereby the present method is executed, and the present apparatus or the present system is realized as a combination of respective units to be described later.

The information processing apparatus 10 (CPU 11) can use a learned model obtained by machine learning using training data.

Here, the "learned model" is a model obtained by machine learning using training data, that is, supervised learning, and can be expressed as an artificial intelligence (AI) model, a machine learning (machine learning (ML)) model, and the like.

The learned model used in the present embodiment may be a regression equation obtained by regression analysis or a neural network model obtained by deep learning or the like. A data structure, a learning algorithm, and the like of the model are not limited. In the present embodiment, a model of a neural network including a convolution operation (CNN) is preferably used as the learned model.

The learned model may be stored in the memory 12 in the information processing apparatus 10, or may be stored in a memory of another computer accessible by the information processing apparatus 10 through communication.

As described above, the information processing apparatus 10 according to the present embodiment is an apparatus capable of using the learned model and is a skin condition estimating apparatus capable of executing the skin condition estimating method.

Hereinafter, the learned model used in the present method is referred to as an AI model or an estimating model. In addition, a group of face images used as the training data in the learning of the AI model is normalized such that the coordinates of the corresponding positions coincide with each other by a common rule as described later, and this rule is referred to as an input rule of the AI model. Furthermore, in the following description, the execution subject of the present method is described as the CPU 11.

### [Skin Condition Estimating Method and Apparatus]

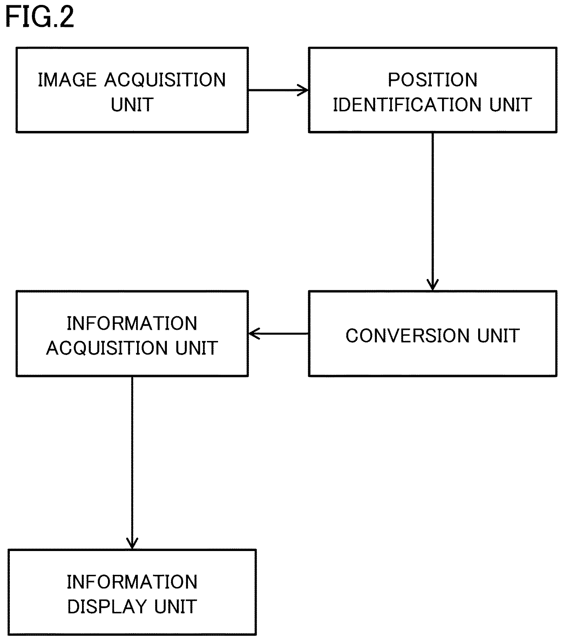

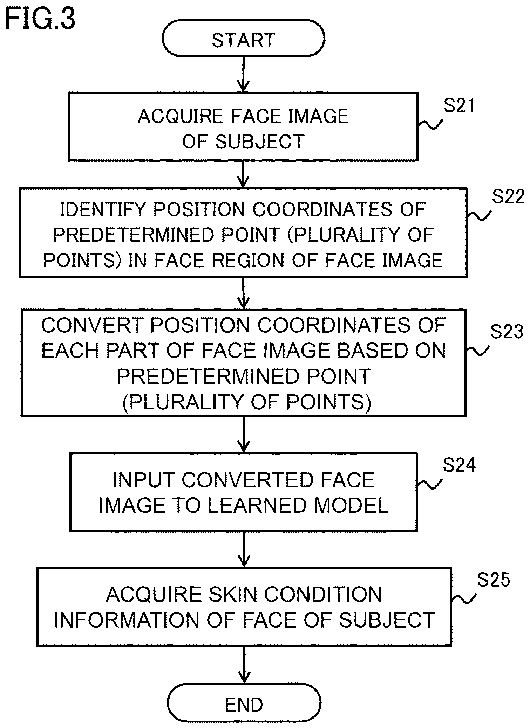

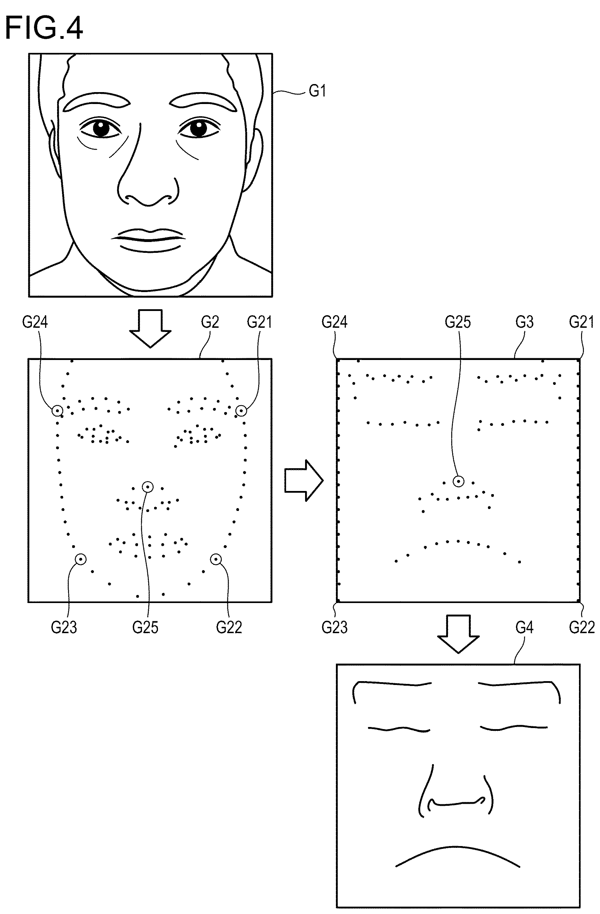

Hereinafter, details of the present embodiment will be described with reference to Figs. 2, 3, and 4. Fig. 2 is a block diagram for explaining functions of respective units of a skin condition estimating apparatus (which may be simply abbreviated as a present apparatus) according to the present embodiment. Fig. 3 is a flowchart illustrating the skin condition estimating method according to the present embodiment, and Fig. 4 is a diagram illustrating a flow of conversion of a face image in the skin condition estimating method according to the present embodiment.

Describing each process of the present method as a functional block, as illustrated in Fig. 2, the present apparatus includes: an image acquisition unit that acquires a face image of a subject; a position identification unit that identifies position coordinates at a plurality of predetermined places in a face region of a human face appearing in the face image acquired by the image acquisition unit; a conversion unit that obtains a converted face image by converting the face image so that a plurality of points on a contour of the face region of the human face appearing in the acquired face image is positioned at an outer edge of an image in order to match an input rule in the AI model; an information acquisition unit that acquires skin condition information of the face of the subject by inputting the converted face image as an input image into the AI model formed by a convolutional neural network prepared in advance; and an information display unit that generates image information for presenting the acquired skin condition information.

The following description of each process also applies to each corresponding unit, unless otherwise identified, and thus, for the operation of each unit, refers thereto.

Each unit is realized by appropriately combining the CPU 11 of the computer, a peripheral circuit, a computer program for realizing each function, a logic circuit, and the like. For example, the unit for executing the skin condition estimating using the AI model is preferably implemented using a GPU, can also be implemented by combining a general CPU and a program. In other words, it is possible to provide a program that causes a computer to function as all or a part of the above skin condition estimating system, and it is also possible to provide a computer-readable recording medium in which such the program is recorded.

The AI model used in the information acquisition unit and an information acquisition process in the present embodiment is a learned model that estimates the skin condition of the human face appearing in the input face image from the input face image having a predetermined image size, that is, a rectangular shape (referred to as a predetermined rectangular shape in the present specification) having a predetermined number of vertical and horizontal pixels. Therefore, the AI model used in the present embodiment is also referred to as a skin condition estimating model.

The "predetermined image size" means a predetermined size of an image and is designated by the number of pixels. The image size is predetermined, for example, 25×20 (500) pixels, 20×20 (400) pixels, 20×11 (220) pixels, and the like. Note that the image size illustrated here is an example.

The "predetermined rectangular shape" means a predetermined rectangular shape of an image and is a rectangle or a square having lengths of long sides and short sides corresponding to the above "predetermined image size".

Hereinafter, in a case where the "predetermined rectangular shape" is written with respect to the face image, it is assumed to indicate a rectangular shape having a predetermined number of vertical and horizontal pixels with respect to the input face image of the AI model.

Note that, although the predetermined rectangular shape is illustrated as an example in the present embodiment, the shape of the input image to the AI model that can be used, that is, a predetermined shape includes a circular shape, an elliptical shape, and the like in addition to the rectangular shape. In any shape, by performing conversion such that coordinates of corresponding positions match according to a common rule, an effect similar to that in a case where the predetermined shape is a rectangle can be obtained.

The information estimated by the AI model may be information indicating the skin condition of the human face, and a specific estimation item is not limited at all. As the estimation items of the skin condition, for example, as exemplified in the examples, makeup feeling, skin age, male/female skin likeness, powdery, glossy, wet, visual wetness, visual dryness, smoothness, fineness, luster, firmness, transparency, skin tone, skin yellowness, dullness, and the like can be estimated. The makeup feeling indicates a feeling that does not look like bare skin, the "powdery" indicates a powdery feeling, and the "glossy" indicates a degree of surface gloss. The estimation items of the AI model are not limited to such items and may be other items such as comprehensive evaluation of beauty.

The AI model may be formed to be able to estimate a plurality of items related to the skin condition of the human face or may be formed to be able to estimate any one of the items. In the former case, for example, the AI model may be constructed by a plurality of neural networks capable of outputting estimation information of different skin condition items. In the latter case, one neural network may be constructed so as to output estimation information of a plurality of skin condition items.

Note that a learning method of the AI model will be described later.

As illustrated in Fig. 3, the present method includes an image acquisition process (S21), a position identification process (S22), a conversion process (S23), and information acquisition processes (S24) and (S25).

In the image acquisition process (S21), the CPU 11 acquires a face image of the subject. In the example of Fig. 4, reference numeral G1 indicates an acquired face image.

The acquired face image only needs to include the face of the subject to the extent that the skin condition of the estimation item can be analyzed, and only needs to include the entire face or a part of the face of the subject. The acquired face image may include a part other than the face of the subject, such as hair, neck, and the like, or background.

Furthermore, the angle and position of the subject appearing in the face image are not particularly limited. For example, the acquired face image may include a front face of the subject or may include a left oblique face or a right oblique face of the subject. Furthermore, a face image obtained by capturing the face of the subject from below or above may be acquired.

The face image acquired in the image acquisition process, that is, the image acquisition unit is acquired as an image file of, for example, a joint photographic experts group (JPEG) format, a bitmap image (BMP) format, a tagged image file format (TIFF) format, a graphic interchange format (GIF) format, and the like.

Furthermore, the acquired face image may be a color image or a grayscale image as long as the skin condition of the human face appearing in the face image can be estimated.

The CPU 11 acquires a face image from a camera that captures the face of the subject, another computer, and the like via a communication line or a portable recording medium.

Hereinafter, the face image acquired in process (S21) may be referred to as an original face image.

Note that the acquired original face image does not need to have the same size as the predetermined image size of the predetermined rectangular shape, but the total number of pixels is preferably larger than the predetermined image size of the predetermined rectangular shape.

Details of the position identification process (S22) will be described.

In the position identification process (S22), first, points on a contour of the entire face region are selected as first contour points, and then the insides of the first contour points are divided into triangular meshes as follows. Then, the vertices of the plurality of meshes are selected as third contour points such that the figure formed by connecting the vertices includes a predetermined local part region, and subsequently, each second contour point is selected between each of the first contour points and each of the third contour points. More specifically, after the face region is divided into a large number of meshes, contour points are selected from points (mesh points) where sides of each mesh intersect. The first contour point is a point located on a contour of the entire face, that is, a boundary between the face and a portion other than the face including the hair and the neck and is preferably a point located slightly inside the boundary. The third contour point is a mesh point formed when the sides of the mesh are connected so that all of the predetermined local part region is wrapped inside them, and the point at which the minimum number of meshes from the first contour point (hereinafter, the minimum number of mesh sides connecting two mesh points is referred to as a mesh distance) is equal to each other is selected as the third contour point. As the second contour point, the mesh point between the first contour point and the third contour point is selected.

After the apparatus automatically generates the meshes and determines the positions of the first contour points on the image, the positions of the third contour points are determined on the image so that the mesh distances from the first contour points are equal, and then the positions of the second contour points on the image are determined from the generated meshes.

When the first to third contour points cannot be set in accordance with the above criteria, for example, when the mesh distance between the first contour point and the third contour point is 1 or less, or when all the third contour points are not equal to the mesh distance from the first contour point, recalculation to make the mesh finer (increase the total number of meshes) is performed, and the position of each mesh point on the image is changed so that the first to third contour points can be selected.

In the position identification process (S22), the CPU 11 identifies position coordinates (particular position coordinates) of a plurality of predetermined points in the face region of the human face appearing in the original face image. In the example of Fig. 4, the points in a rectangle denoted by reference numeral G2 indicate the position coordinates of a plurality of identified positions.

The position coordinates identified in process (S22) include at least the position coordinates of the shape feature point of the face region. The shape feature point of the face region indicates a point at a position having the shape feature of the human face, and corresponds to, for example, a point on the contour of the face, a point on the contour of the eye (the upper eyelid edge and the lower eyelid edge), a point on the contour of the eyebrow, a point on the contour of the lip (the upper lip edge and the lower lip edge), the vertex of the nose (the tip of the nose), a point on the ridge of the nose (the bridge of the nose), a point of the nose root, and the like. However, the position coordinates identified in process (S22) are not limited to the position coordinates of such a shape feature point, and may be position coordinates of a predetermined point in the face region derived from such a shape feature point, or may include the position coordinates of the shape feature point and position coordinates of other points.

Note that the position identification process (S22) is executed by the CPU 11, and a user can sequentially identify the original face image displayed on the display device according to the instruction. In this case, the position may be identified by a pointing device such as a mouse, but in terms of operability, it is preferable to use a touch screen. In a so-called smartphone terminal, a touch screen is used. It is needless to say that it is preferable to automatically perform the operation using a computer because the operation performed by the user is complicated.

The particular position coordinates identified in process (S22) preferably include position coordinates of a plurality of outer peripheral points surrounding the predetermined local part region in the face region.

The "predetermined local part region" is a region of a part that should preferably be removed from the face region in the face image input to the AI model because it does not contribute to estimation of the skin condition. For examples, the part is eyes, eyebrows, lips, nostrils, whiskers, and chin whiskers.

As a method of removing the pixel of the predetermined local part region, which is preferably removed, from the face region in the face image input to the AI model, the present embodiment performs the following method. The pixel of the skin surrounding the predetermined local part region is arranged on a straight line or a curve in the face model, and as a result, the pixel in the predetermined local part region is not projected to the face model. At this time, the positions of the outer peripheral points facing each other in the predetermined local part region coincide with each other on the face model.

In the present embodiment, the "predetermined local part region" is an eye region, an eyebrow region, and a lip region. The lip region is an upper lip region and a lower lip region, in addition to these, the lip region includes a mouth region in a case where the subject appearing in the face image is in a state of opening the mouth.

The plurality of outer peripheral points may be points located so as to surround the predetermined local part region in the face region and may be points on the contour of the predetermined local part region or points outside the contour of the predetermined local part region.

As described above, the position coordinates of the plurality of outer peripheral points surrounding the predetermined local part region in the face region are identified in process (S22), whereby the predetermined local part region can be removed in conversion process (S23) to be described later.

Although it is preferable that the predetermined local part region be completely removed, even a reduced form can contribute to improvement of estimation accuracy. For example, when points on the contour of the predetermined local part region or points slightly inside the contour are identified as the plurality of outer peripheral points, and the identified outer peripheral points are arranged on a straight line or a curve in the face model, the predetermined local part region is reduced and remains. Even in such a case, since the region that does not contribute to the estimation of the skin condition can be reduced, the estimation accuracy of the skin condition by the AI model can be improved.

In addition, it is preferable that the particular position coordinates identified in the process (S22) include position coordinates of a plurality of points on the contour of the face region, that is, on the boundary between the face region and other regions, a nose vertex inside the face region, and a plurality of other points inside the face region.

In the conversion process (S23) described later, four first contour points among the points on the contour of the face region are arranged at vertices of a predetermined rectangular shape of the face image, and the other points on the contour of the face region are arranged on sides of the predetermined rectangular shape of the face image. Therefore, the four first contour points are set to two upper points on the left and right on the contour of the face region and two lower points on the left and right on the contour.

In the example of Fig. 4, two points (G21 and G24) on the contour adjacent to the ends of the left and right eyebrows and two points (G22 and G23) on the contour adjacent to the right and left corners of the mouth correspond to the first contour points, and the nose vertex is indicated by reference numeral G25. However, the first contour points may be a combination of two upper left and right points on the contour of the face region and two lower left and right points on the contour and are not limited to the example of Fig. 4.

As a method of identifying position coordinates in the process (S22), face recognition process that has been used in various scenes can be used. In the face recognition process, for example, an AI model capable of detecting the positions of a plurality of nodes of a human face such as Facemark of Open Source Computer Vision Library (OpenCV) or FaceMesh of Google (registered trademark) is used. In the present embodiment, the method for identifying the position coordinates in the process (S22) is not limited at all. For example, in process (S22), the CPU 11 can identify position coordinates of some or all of the landmarks detected using such a face recognition AI model as position coordinates of a plurality of points in the face region.

In the conversion process (S23), the CPU 11 performs a so-called mapping operation to convert the position of each pixel of the original face image acquired in the process (S22) so that the face region has a predetermined rectangular shape by coordinate-converting the position coordinates of a plurality of predetermined points as a reference identified in the process (S21) and the position of each pixel of the original face image becomes a position corresponding to the input rule of the AI model. That is, the conversion process (S23) can also be referred to as a coordinate conversion process or a mapping process. Similarly, the conversion unit described later can also be referred to as a coordinate conversion unit or a mapping unit.

In the example of Fig. 4, the position coordinates after the coordinate conversion are indicated in a rectangle indicated by reference numeral G3, and reference numeral G4 indicates the face image after the conversion. In other words, each pixel converts only its position while maintaining its value. When the position of each pixel changes, the spatial distance between the pixels changes. Therefore, operations are performed such as, when the distance increases, the value of the pixel filling the space is obtained by interpolation, and when the distance decreases, the value of a new pixel is obtained by using, for example, an average value. As a specific method, it is sufficient to apply a well-known technique such as a morphing technique, and thus, details thereof will not be repeated. Note that position coordinates of predetermined points serving as a reference of the original face image may coincide with the position corresponding to the input rule of the AI model by chance, and in such a case as well, a person skilled in the art will naturally understand that the position coordinates are included in targets of the mapping operation or the position conversion.

The method is not limited as long as the coordinate conversion is performed such that the face region of the original face image has a predetermined rectangular shape.

Note that the position identification process (S22) and the conversion process (S23) can be realized as one conversion process. That is, if it is determined which position of a reference image a certain pixel of the original face image corresponds to, the coordinates of the conversion destination (projection destination) can be immediately identified, and thus, it is not always necessary to perform the process in two processes. This also applies to the position identification unit and the conversion unit, and in the present apparatus, the position identification unit and the conversion unit can be configured as an integrated conversion unit.

In this manner, by converting the original face image into a face image having a predetermined rectangular shape and in which the face region occupies the entire face image, it is possible to eliminate a region other than the face region and enlarge the face region in the face image input to the AI model. As a result, it is possible to prevent a reduction in the resolution of the face region in the face image input to the AI model, and to improve the estimation accuracy of the AI model.

Such an operation and effect becomes remarkable when the AI model is realized by the neural network model including the convolution operation. In such an AI model, since the convolution operation is repeated in a state where information other than the face region of the input image is stored, the influence of the region (region other than a skin region) that is not the analysis target included in the input image remains even in the deep layer of the AI model. As a result, unnecessary information remains in the AI model. Furthermore, as a result of including information other than the face region, the information to be analyzed is reduced. These factors lead to a decrease in the estimation accuracy of the AI model.

When position coordinates of a plurality of points on the contour of the face region, the nose vertex of the face region and a plurality of other points of the face region are identified in process (S22), coordinate conversion in process (S23) is performed as follows. That is, as illustrated in the example of Fig. 4, each of the four first contour points (G21, G22, G23 and G24) included in the plurality of points on the contour of the face region of the original face image is arranged at the vertex of the predetermined rectangular shape, the nose vertex (G25) is arranged at the center of the predetermined rectangular shape, and the coordinate conversion is performed such that the plurality of other points of the face region moves corresponding to the movement of the nose vertex and the first contour points.

By performing the coordinate conversion in this manner, the face region can occupy the entire face image of the converted predetermined rectangular shape. Furthermore, according to the coordinate conversion, it is possible to make the shape and size of the contour of the face of each individual appearing in the original face image uniform without a difference. As a result, since the positions of the nose, the mouth, the eyes, and the like in the face region can be substantially standardized in the face image input to the AI model, the learning efficiency and the like of the AI model can be improved, and the estimation accuracy of the AI model can be improved.

In the present embodiment, since the position coordinates of the plurality of outer peripheral points surrounding the predetermined local part region in the face region are identified in process (S22), the image conversion in process (S23) is performed as follows. That is, the CPU 11 matches the position coordinates of the outer peripheral points facing each other via the predetermined local part region in the coordinate conversion, in other words, connects the outer peripheral points, and excludes the predetermined local part region in the face region of the original face image.

Here, the "connection of the outer peripheral points" may be realized by coordinate-converting two or more outer peripheral points facing each other via the predetermined local part region into the same position coordinates, or may be realized by moving the two or more outer peripheral points to positions close to each other so that the local part region disappears or is reduced.

As a result, it is possible to enlarge the skin region in the face image while excluding the local part region irrelevant to the skin condition of the human face from the face image input to the AI model. As a result, it is possible to prevent a reduction in the resolution of the face region, which occurs when the number of pixels of the face region of the original face image is smaller than the predetermined rectangular shape of the input image of the AI model, as compared with the conventional method, and eventually, it is possible to improve the estimation accuracy of the AI model.

For this reason, the predetermined local part region is preferably any one of the eye region, the eyebrow region, or the lip region irrelevant to the skin condition, more preferably any ones of the eye region, the eyebrow region, or the lip region, and further preferably all of them.

In the information acquisition processes (S24) and (S25), the CPU 11 inputs the face image converted in the process (S23) to the AI model (S24) and acquires the skin condition information of the face of the subject (S25).

In a case where the program or the like for realizing the AI model is stored in the memory 12 in the information processing apparatus 10, the CPU 11 can acquire the skin condition information of the face of the subject as the output of the AI model by inputting the face image converted in the process (S23) to the AI model.

As described above, the skin condition information acquired in the process (S25) may be information indicating the skin condition of the face of the subject, and specific estimation items are not limited at all. In a case where the AI model is formed so as to be able to estimate a plurality of items related to the skin condition of the human face, the acquired skin condition information may be information of a plurality of skin condition items or may be information of one skin condition item.

In addition, in recent years, there are various techniques indicating the basis of determination by the AI model. For example, there is a technique called GradCam for an AI model of CNN, and it is possible to know which region contributes to the conclusion.

Therefore, in the process (S23), coordinate conversion may be performed using such a technique so that the part of the face region that contributes to the estimation result of the AI model is further weighted. Alternatively, in the process (S25), the CPU 11 can acquire the skin condition information of the face of the subject and the information on the position of the part of the face region corresponding to the skin condition information. At this time, the CPU 11 can also output, for example, a face image in which the part in the face region of the original face image is identifiably displayed. By providing the process of displaying the part contributing to the skin condition on the screen in this manner, this is preferable because it is possible to display an image that can easily understand which part of the subject should be applied with the operation or the product.

Furthermore, the CPU 11 can further execute a process of outputting the recommendation information of one or both of the beauty treatment method or the beauty product corresponding to the skin condition of the face of the subject based on the skin condition information acquired in the process (S25). For example, the output recommendation information includes information of a beauty treatment method or a beauty product such as cosmetics or supplements recommended for improving the skin condition of the subject or achieving the skin age desired by the subject. At this time, as described above, in a case where the part information of the face region corresponding to the skin condition information can be acquired, the output recommendation information can present the beauty product or the beauty treatment method different for each part of the face region. Furthermore, in a case where the AI model is formed so as to be able to estimate a plurality of skin condition items, the output recommendation information can present different beauty products or beauty treatment methods for each skin condition item.

Each of the plurality of types of beauty treatment methods and the plurality of types of beauty products is stored in the memory 12 in association with each skin condition (each skin condition of each skin condition item) that can be estimated by the AI model, and the CPU 11 can specify one or more beauty treatment methods or one or more beauty products corresponding to the skin condition information acquired in the process (S25) on the basis of the information stored in the memory 12, and generate recommendation information including one or both of the identified methods or products. The association information between the method/product and the skin condition may be stored in a storage device of another computer and referred to via communication.

The output form of the recommendation information is not particularly limited. For example, the CPU 11 may cause the display device 15 to display the recommendation information, may cause a printing device to print the recommendation information, may transmit the recommendation information to another computer, or may store the recommendation information as an electronic file in a portable recording medium or the like.

### [Learning Method of AI Model (Skin Condition Estimating Model)]

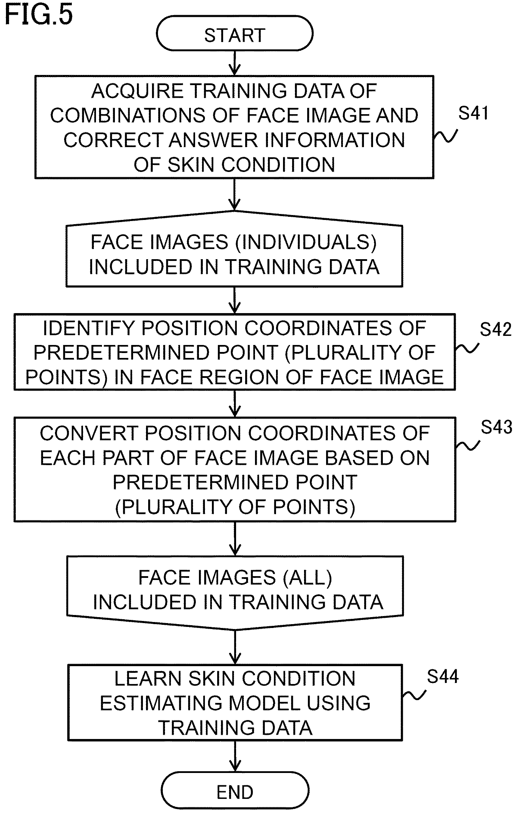

A learning method of the AI model (skin condition estimating model) used in the above-described present method will be described with reference to Fig. 5.

Fig. 5 is a flowchart illustrating a learning method (hereinafter, it may be referred to as the present learning method) of the skin condition estimating model according to the present embodiment. The present learning method can be executed by the information processing apparatus 10 (CPU 11) as illustrated in Fig. 1.

The present learning method generally includes a process (S41) of acquiring training data, a conversion process (S42) (S43) of converting each face image included in the training data, and a process (S44) of causing the AI model to learn using the training data including the converted face image. Hereinafter, each process of the present learning method will be described in detail.

In the process (S41), the CPU 11 acquires the training data including a plurality of combinations of the face image and the correct answer information indicating the skin condition of the human face appearing in the face image.

Each face image included in the training data is as described with respect to the face image acquired in the process (S21) of the present method, and it is sufficient that the face of the subject is reflected to such an extent that the skin condition of the estimation item can be analyzed. Each face image included in the training data may be normalized to a predetermined rectangular shape or may include a face image having an image size different from the predetermined image size of the predetermined rectangular shape or a shape different from the predetermined rectangular shape.

The correct answer information included in the training data is information indicating the skin condition of the human face obtained by the evaluator visually or evaluating the corresponding face image or the subject itself of the face image using a measuring instrument or the like. In a case where the AI model is formed so as to be able to estimate a plurality of items related to the skin condition of the human face, the correct answer information is information of a plurality of skin condition items.

Process (S42) and process (S43) are executed for each face image included in the training data obtained in process (S41).

In process (S42), the CPU 11 identifies position coordinates (particular position coordinates) of a plurality of predetermined points in the face region of the human face appearing in the target face image.

In process (S43), the CPU 11 coordinate-converts the target face image such that the face region has a predetermined rectangular shape based on the position coordinates of the plurality of predetermined points identified in process (S42).

The processing contents of process (S42) and process (S43) are the same as those of processes (S22) and (S23) described above.

The process (S42) and the process (S43) are executed for all the face images included in the training data, then the CPU 11 causes the AI model to learn using the training data including the converted face image in the process (S44). In the present embodiment, the AI model is learned by deep learning. However, a specific learning algorithm for the AI model is not limited at all.

As described above, in the present learning method, the AI model is learned using the face image in which the face region occupies the entire face image as the training data. That is, the face image used for learning the AI model is an image in which at least a plurality of points on the contour of the face region is located at an outer edge of the image. It is more preferable that the face image is an image in which all points on the contour of the face region are located at the outer edge of the image.

As a result, the face image excluding the image region other than the face region is used as the training data, and the learning process of learning that the image region other than the face region is not used for estimation can be omitted, so that the learning processing of the AI model can be made efficient. Furthermore, in the face image used as the training data in the present learning method, since the face region is expanded by the number of pixels of the image region excluding the face region, it is possible to suppress a decrease in the resolution of the face region to be estimated, and eventually, it is possible to construct a highly accurate AI model.

Furthermore, the face image used for learning the AI model is more preferably an image in which a predetermined local part region in the face region is removed or reduced, and is more preferably a face image in which a plurality of points (including the first contour point) on the contour of the face region, the nose vertex inside the face region, and a plurality of other points (including the second contour point and the third contour point) inside the face region are coordinate-converted as described in the present method.

The face image used for learning of the AI model is subjected to coordinate conversion (normalization) such that the coordinates of the corresponding positions match by such a common rule.

### [Modification]

The contents of the above-described embodiment can be appropriately modified within a range in which there is no problem.

For example, in the example of the AI model described above, learning is performed using a face image having a predetermined rectangular shape as training data, but learning may be performed using a face image having a non-rectangular shape such as a circular shape or an elliptical shape as training data. Even in this case, in the conversion process (S23), the CPU 11 may convert the original face image such that the plurality of points on the contour of the face region of the human face appearing in the original face image is positioned at the outer edge of the image in order to match the input rule in the AI model, that is, such that the face region occupies the whole of the face image having a predetermined shape other than a rectangle to obtain the converted face image.

Furthermore, in the above-described embodiment, as a preferable aspect, the processing of removing or reducing the predetermined local part region in the face region is performed in the conversion process (S23), but this processing may be omitted.

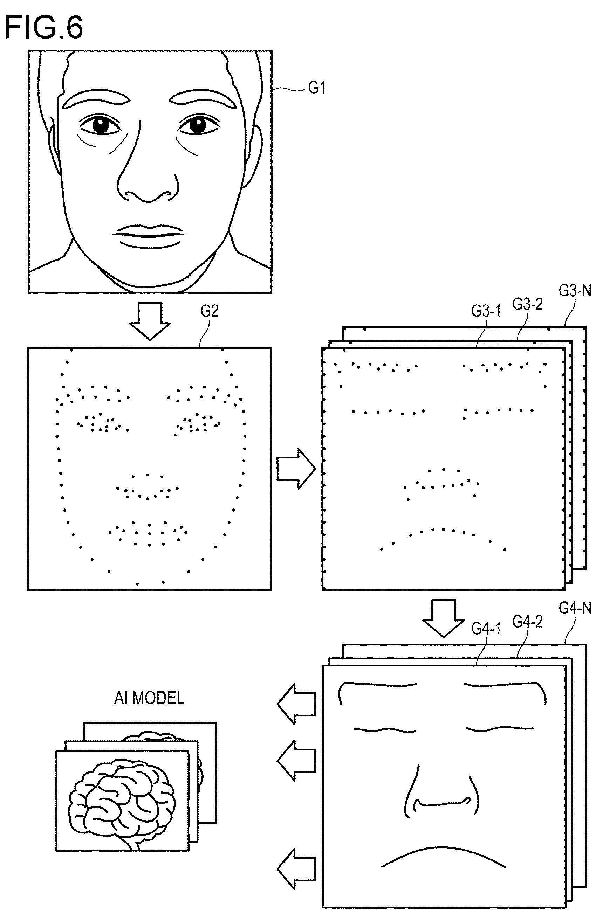

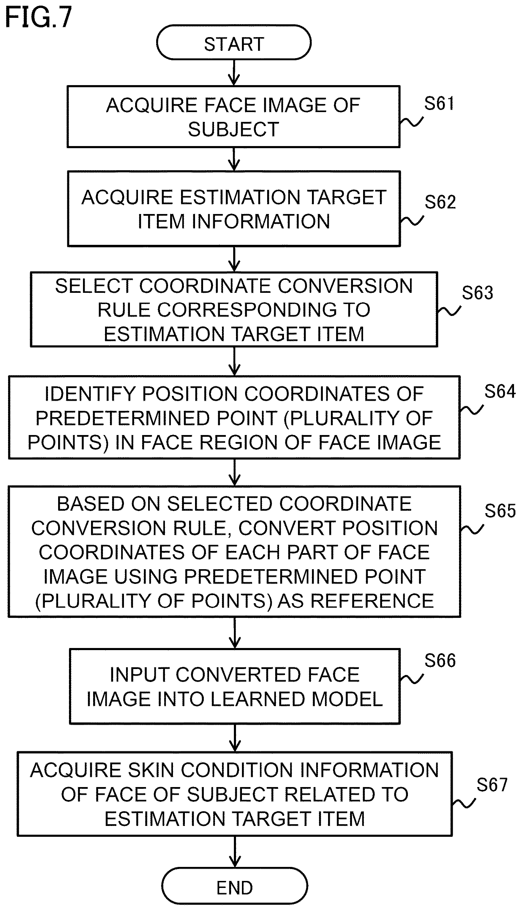

In a case where the AI model is formed so as to be able to estimate a plurality of items of the skin condition, the above-described embodiment can be modified as illustrated in Figs. 6 and 7.

Fig. 6 is a diagram illustrating a flow of conversion of the face image in the skin condition estimating method (hereinafter, it is referred to as the present modification method) according to the modification, and Fig. 7 is a flowchart illustrating the skin condition estimating method according to the modification.

The AI model used in the present modification method is formed by, for example, a plurality of neural networks capable of outputting estimation information of one or more skin condition items different from each other. Each neural network is learned by using training data including a combination of the face image converted to match an input rule corresponding to one or more skin condition items to be estimated and correct answer information of the skin condition item. Therefore, it can be said that a plurality of the input rules is provided in the AI model used in the present modification method.

The CPU 11 can use the plurality of coordinate conversion rules different from each other corresponding to a plurality of estimation items for obtaining a face image conforming to the input rule of the AI model.

The coordinate conversion rule is used for coordinate conversion in process (S65) described later and defines a position to which position coordinates of a plurality of predetermined points identified in process (S64) described later are moved in the converted face image.

As described in the above embodiment, the coordinate conversion rule may be any rule as long as the original face image can be converted such that the face region occupies the entire face image after the conversion, and the specific content is not limited at all. For example, the coordinate conversion rule is set such that a part region corresponding to the skin condition item to be estimated in the skin region of the face is enlarged, and a part region having a low relationship with the skin condition item is reduced. Specifically, the coordinate conversion rule corresponding to the skin condition item of skin age is defined so as to enlarge the region of the outer corner of the eye or a nasolabial region. In addition, the coordinate conversion rule corresponding to the dry skin condition item is defined to expand an eye region and a mouth region known to be easily dried in the face skin, and the coordinate conversion rule corresponding to the glossy skin condition item is defined to expand a region called a T zone known as a point having a large amount of sebum.

The number of coordinate conversion rules may be the same as the number of items that can be estimated by the AI model or may be smaller than the number of items that can be estimated by the AI model. In the former case, one coordinate conversion rule is provided for one estimation item, and a neural network is formed for each coordinate conversion rule, in other words, for each estimation item. In the latter case, one coordinate conversion rule provided for a plurality of estimation items and one coordinate conversion rule provided for one estimation item may be mixed, or a plurality of coordinate conversion rules provided for a plurality of estimation items may be provided. Also in this case, a neural network is formed for each coordinate conversion rule, but not for each estimation item.

As illustrated in Fig. 7, the present modification method includes processes (S61) to (S67).

The process (S61) is similar to the process (S21) described above.

In process (S62), the CPU 11 acquires estimation target item information. The estimation target item information is information indicating one or more requested skin condition items among the plurality of skin condition items that can be estimated by the AI model. The CPU 11 can acquire the estimation target item information according to the input of the user using the input device 16, can acquire the preset estimation target item information from the memory 12, and can acquire the estimation target item information via communication from another computer.

In process (S63), the CPU 11 selects a coordinate conversion rule corresponding to the estimation target item indicated by the estimation target item information acquired in process (S62). At this time, in a case where a plurality of items is designated as the estimation target item, one or more coordinate conversion rules corresponding to the plurality of items are selected.

The process (S64) is similar to the process (S22) described above.

In process (S65), the CPU 11 converts the original face image by coordinate-converting the position coordinates of the plurality of points identified in process (S64) based on the coordinate conversion rule selected in process (S63) and generates the converted face image corresponding to the estimation target item from the original face image. The face image conversion method in process (S65) is similar to that in process (S23) except that a coordinate conversion rule selected from a plurality of coordinate conversion rules are used.

In a case where a plurality of coordinate conversion rules is selected in process (S63), a plurality of converted face images respectively corresponding to a plurality of estimation target items is generated in process (S65). In the example of Fig. 6, N (G3-1, G3-2, ..., G3-N) position coordinates are indicated as position coordinates after coordinate conversion based on the N coordinate conversion rules, and N converted face images (G4-1, G4-2, ..., G4-N) are generated.

Process (S66) is similar to process (S24) described above, and process (S67) is similar to process (S25) described above.

In a case where a plurality of items is designated as the estimation target item, the skin condition information regarding the plurality of items of the face of the subject are acquired in process (S67). Furthermore, in a case where a plurality of coordinate conversion rules is selected in the process (S63) and a plurality of converted face images is generated in the process (S65), the plurality of converted face images is input to the AI model in the process (S66), and skin condition information regarding a plurality of items of the face of the subject are acquired in the process (S67).

According to the present modification method, the skin condition information of the subject regarding a plurality of items can be acquired. Furthermore, the skin condition information of the subject regarding a desired item among the plurality of items can be acquired.

In addition, since the AI model performs estimation on the basis of the face image converted using the coordinate conversion rule corresponding to the skin condition item for each skin condition item to be estimated, the estimation accuracy for each estimation item of the skin condition can be improved.

The present modification method can be further modified. For example, it is also possible to omit the process (S62) of acquiring the estimation target item information from the present modification method and perform deformation such that the skin condition information regarding all the estimation items that can be estimated by the AI model is acquired.

### [Other Embodiments]

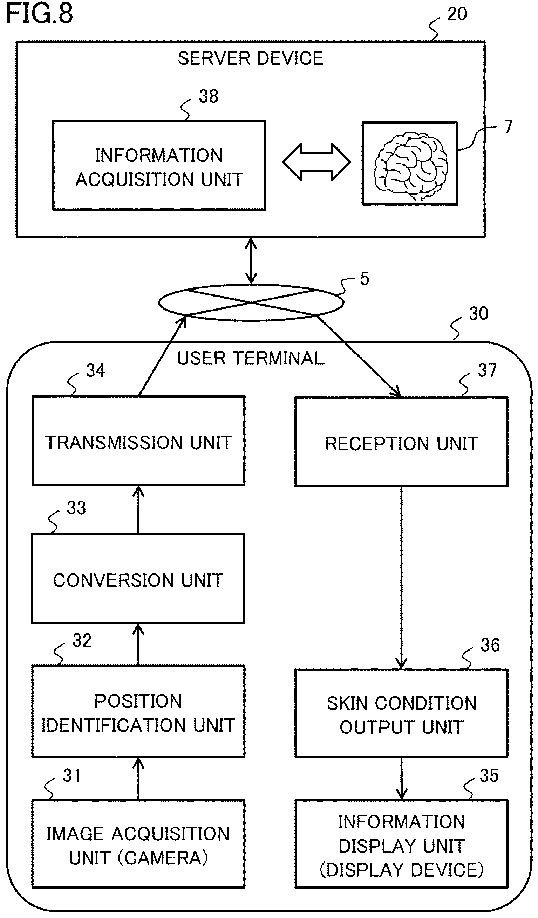

Fig. 8 illustrates a configuration of another embodiment (hereinafter, the present embodiment) of the skin condition estimating system according to the present invention. In the present embodiment, a user terminal 30 such as a smartphone and a server device 20 are connected via a network to constitute a system.

In the present embodiment, the server device 20 includes an AI model 7 and an information acquisition unit 38. The user terminal 30 includes a camera 31 that is the image acquisition unit, a position identification unit 32, a conversion unit 33, a transmission unit 34, an information display unit 35 that is a display device, a skin condition output unit 36 that converts-and-generates skin information presented in the information display unit 35 into image information that can be read by a user, and a reception unit 37.

The user terminal 30 and the server device 20 are connected via a communication line 5 such as the Internet, and integrally constitute the skin condition estimating system. Note that a plurality of user terminals 30 may be provided, and in this case, a person skilled in the art can easily understand that each combination of each user terminal 30 and the server device 20 constitutes the skin condition estimating system.

The converted face image transmitted to the server device 20 by the transmission unit 34 of the user terminal 30 is received by a reception unit (not shown in the drawings) of the server device 20 and input to the AI model 7 via the information acquisition unit 38. In the present embodiment, the skin condition information output from the AI model 7 and acquired by the information acquisition unit 38 is transmitted from the transmission unit (not shown in the drawings) of the server device 20 to the user terminal 30 via the communication line 5. The skin condition information received by the reception unit 37 of the user terminal 30 is converted into data constituting a display screen by the skin condition output unit 36 and is displayed on the display device 35 (screen) of the user terminal 30.

As described above, by arranging the AI model 7 in the server device 20, the AI model 7 can be replaced or updated without changing the computer program on the user terminal 30, accuracy can be improved, and the frequency of accuracy improvement can be increased as the entire system.

It is preferable that a program, that is, a program for causing the user terminal 30 to function as the position identification unit 32, the conversion unit 33, the transmission unit 34, the information display unit 35 which is a display device, the skin condition output unit 36 which converts and generates skin information presented in the information display unit 35 into image information readable by the user, and the reception unit 37 in the present system may be transmitted to the user terminal 30 via a communication medium forming a network. The program may be stored in a storage medium and provided to the user of the user terminal 30.

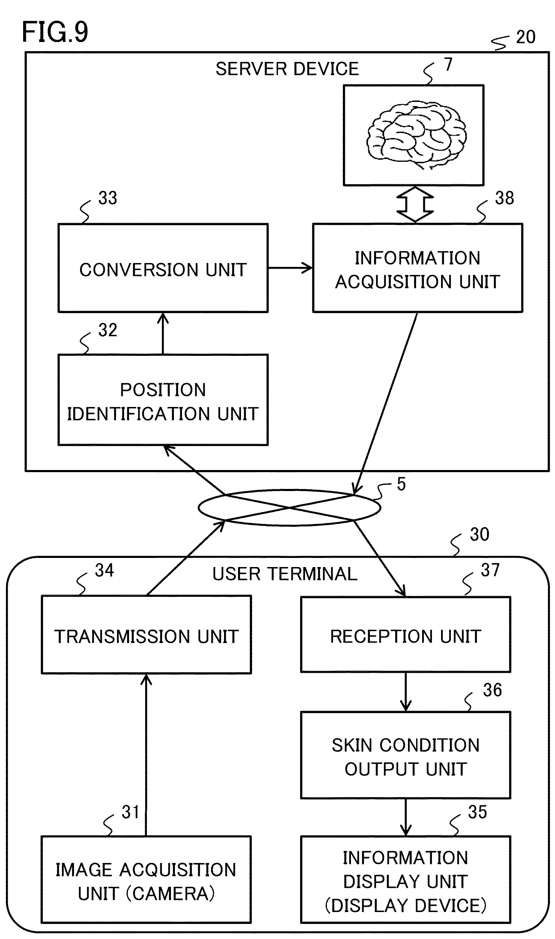

Fig. 9 illustrates a configuration of still another embodiment (hereinafter, the present embodiment) of the skin condition estimating system according to the present invention. In the present embodiment, as in the example illustrated in Fig. 8, the user terminal 30 such as a smartphone and the server device 20 are connected via a network to constitute a system.

The user terminal 30 includes the camera 31 that is the image acquisition unit, the transmission unit 34, the information display unit 35 that is a display device, the skin condition output unit 36, and the reception unit 37 that receives the skin condition. The server device 20 includes the AI model 7, the position identification unit 32, the conversion unit 33, and the information acquisition unit 38.

Similarly to the skin condition estimating system illustrated in Fig. 8, the user terminal 30 and the server device 20 are connected via the communication line 5 such as the Internet, and integrally constitute a skin condition estimating system. Note that a plurality of user terminals 30 may be provided, and in this case, a person skilled in the art can easily understand that each combination of each user terminal 30 and the server device 20 constitutes the skin condition estimating system.

It is preferable that a program for causing the user terminal 30 to function as the transmission unit 34, the skin condition output unit 36, and the reception unit 37 for receiving the skin condition in the present system is transmitted to each user terminal 30 via a communication medium forming a network. The program may be stored in the storage medium and provided to the user of the user terminal 30.

The original face image captured by the camera 31 of the user terminal 30 is transmitted to the server device 20 by the transmission unit 34, processed by the position identification unit 32 and the conversion unit 33 in the server device 20, and then input to the AI model 7 via the information acquisition unit 38.

In the present embodiment, the skin condition information output from the AI model 7 is acquired by the information acquisition unit 38 of the server device 20 and is transmitted to the user terminal 30 via the communication line 5 by the transmission unit (not shown in the drawings). The skin condition information received by the reception unit 37 of the user terminal 30 is converted into data constituting a display screen by the skin condition output unit 36 and is displayed on the display device 35 (screen) of the user terminal 30.

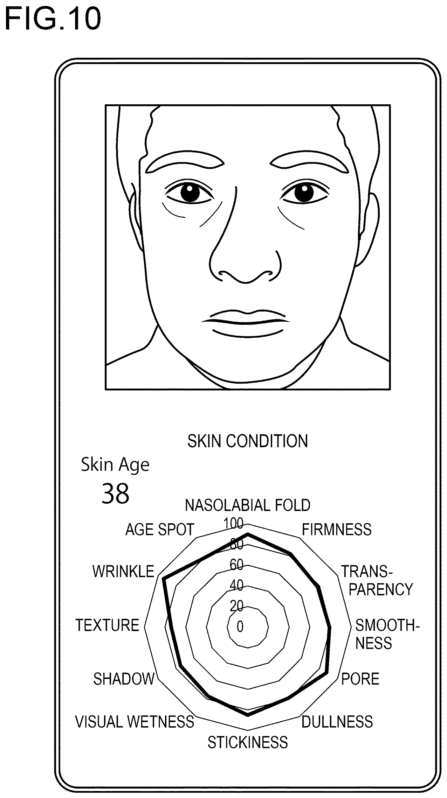

Fig. 10 illustrates an example of a display screen processed and created by the skin condition output unit 36 and displayed on the display device 35 of the user terminal 30. In this display screen, the original face image is displayed in the upper part of the screen, the skin age is indicated by a number in the vicinity of the center of the screen, and the value for each item is indicated by a radar chart in the lower part.

In this manner, by performing the information processing by the skin condition output unit 36 so that the original image and the graph indicating the skin condition information are shown on the same screen, it is possible to obtain a screen on which the skin condition can be easily grasped. Alternatively, by performing the information processing by the output unit 36 in order to display a plurality of pieces of skin condition information on one screen at a time, it is possible to display the entire image of the skin condition at a time.

Although the numerical information transmitted from the server device 20 can be displayed as a simple number, it is important as a communication tool with the user to process and display the numerical information in an easily viewable manner by the skin condition output unit 36 of the user terminal 30 in this manner. When the display screen is generated by the server device 20, in the case of a different user terminal 30, there is a possibility that an adverse effect such as a part of the screen being missing or characters being difficult to read may occur. Therefore, it is preferable that the skin condition information transmitted from the server device 20 be converted for display on the user terminal 30 side as in the present embodiment. In addition, as in the present embodiment, it is preferable that each item of the skin condition is processed into a graph and displayed, and the comprehensive evaluation is indicated by a numerical value such as a skin age, or for example, a score on a scale of 5 or 100 points.

Some or all of the above-described embodiments and modifications can also be identified as follows. However, the above-described embodiments and modifications are not limited to the following description.

<1> A skin condition estimating method using an estimating model learned to output a skin condition of a human face appearing in an input face image from the input face image, the method in which
   one or more processors execute:
   an image acquisition process of acquiring a face image of a subject;
   a conversion process of converting the face image so that a plurality of points on a contour of the face region of the human face appearing in the acquired face image is positioned at an outer edge of an image in order to match an input rule in the estimating model to obtain a converted face image; and
   an information acquisition process of acquiring skin condition information of the face of the subject output from the estimating model by inputting the converted face image as the input face image.
<2> The skin condition estimating method according to <1>, in which
   in the converted face image, a predetermined local part region in a face region of a human face appearing in the acquired face image is removed or reduced, and
   in the conversion process, positions of outer peripheral points facing each other via the predetermined local part region among a plurality of outer peripheral points surrounding the predetermined local part region in the face region are matched, and the predetermined local part region in the face region is removed or reduced.
<3> The skin condition estimating method according to <2>, in which
   the predetermined local part region includes an eye region, an eyebrow region, and a lip region.
<4> The skin condition estimating method according to <2> or <3>, in which
   the one or more processors further execute a position identification process of identifying position coordinates of a plurality of predetermined points in the face region of the human face appearing in the acquired face image, and
   in the conversion process, the acquired face image is converted so as to conform to the input rule of the estimating model by coordinate-converting some or all of the identified position coordinates of the plurality of predetermined points.
<5> The skin condition estimating method according to <4>, in which
   the input face image has a predetermined rectangular shape,
   the position coordinates of the plurality of predetermined points further include position coordinates of the plurality of points on the contour of the face region, the nose vertex of the face region, and a plurality of other points of the face region, and
   in the conversion process, the coordinate conversion is performed such that each of four first contour points included in the plurality of points on the contour of the face region is arranged at a vertex of the predetermined rectangular shape, the nose vertex is arranged at a center of the predetermined rectangular shape, another point on the contour of the face region is arranged on a side of the predetermined rectangular shape, and the plurality of other points of the face region moves corresponding to the movement of the nose vertex and the plurality of points on the contour of the face region.
<6> The skin condition estimating method according to <5>, in which
   the plurality of other points of the face region includes four third contour points located closer to the contour of the face region than the predetermined local part region and four second contour points located further closer to the contour of the face region than the four third contour points, and
   in the conversion process, the four second contour points and the four third contour points are respectively arranged on diagonal lines connecting the four first contour points and the nose vertex, and the coordinate conversion is performed such that a difference between a distance of the first contour point from the nose vertex and a distance of the second contour point from the nose vertex is smaller than a difference between a distance of the second contour point from the nose vertex and a distance of the third contour point from the nose vertex.
<7> The skin condition estimating method according to any one of <1> to <6>, in which,
   the estimating model is capable of estimating a plurality of items of the skin condition,
   a plurality of the input rules of the estimating model is provided,
   in the conversion process, the acquired face image is converted so as to correspond to the input rule corresponding to an estimation target item in the plurality of input rules different from each other corresponding to the plurality of items, thereby acquiring a converted face image corresponding to the estimation target item, and
   in the information acquisition process, the converted face image is input to the estimating model to acquire skin condition information of the subject regarding the estimation target item.
<8>
   A skin condition estimating system includes:
   an estimating model learned to output a skin condition of a human face appearing in an input face image from the input face image;
   an image acquisition unit configured to acquire a face image of a subject;
   a conversion unit configured to obtain a converted face image by converting the face image so that a plurality of points on a contour of a face region of the human face appearing in the acquired face image is positioned at an outer edge of an image in order to match an input rule in the estimating model;
   an information acquisition unit configured to input the converted face image to the estimating model as the input face image and acquire skin condition information of the face of the subject output from the estimation model;
   a skin condition output unit configured to generate image information for presenting the acquired skin condition information; and
   an information display unit configured to display the image information.
<9> The skin condition estimating system according to <8>, in which
   in the converted face image, a predetermined local part region in a face region of a human face appearing in the acquired face image is removed or reduced, and
   the conversion unit matches positions of outer peripheral points facing each other via the predetermined local part region among a plurality of outer peripheral points surrounding the predetermined local part region in the face region to remove or reduce the predetermined local part region in the face region.
<10> The skin condition estimating system according to <9>, in which
   the predetermined local part region includes an eye region, an eyebrow region, and a lip region.
<11> The skin condition estimating system according to <9> or <10>, further including a position identification unit configured to identify position coordinates of a plurality of predetermined points in the face region of the human face appearing in the acquired face image, wherein
   the conversion unit coordinate-converts some or all of the position coordinates of the plurality of predetermined identified points to convert the acquired face image so as to match the input rule of the estimating model.
<12> The skin condition estimating system according to <11>, in which
   the input face image has a predetermined rectangular shape,
   the position coordinates of the plurality of predetermined points further include position coordinates of the plurality of points on the contour of the face region, the nose vertex of the face region, and a plurality of other points of the face region, and
   the conversion unit performs the coordinate conversion such that each of four first contour points included in the plurality of points on the contour of the face region is arranged at a vertex of the predetermined rectangular shape, the nose vertex is arranged at a center of the predetermined rectangular shape, another point on the contour of the face region is arranged on a side of the predetermined rectangular shape, and the plurality of other points of the face region moves corresponding to the movement of the nose vertex and the plurality of points on the contour of the face region.
<13> The skin condition estimating system according to <12>, in which
   the plurality of other points of the face region includes four third contour points located closer to the contour of the face region than the predetermined local part region and four second contour points located further closer to the contour of the face region than the four third contour points, and
   the conversion unit performs the coordinate conversion such that the four second contour points and the four third contour points are respectively arranged on diagonal lines connecting the four first contour points and the nose vertex, and a difference between a distance of the first contour point from the nose vertex and a distance of the second contour point from the nose vertex is smaller than a difference between a distance of the second contour point from the nose vertex and a distance of the third contour point from the nose vertex.
<14> The skin condition estimating system according to any one of <8> to <13>, in which
   the estimating model is capable of estimating a plurality of items of the skin condition,
   a plurality of the input rules of the estimation model is provided,
   the conversion unit converts the acquired face image so as to correspond to the input rule corresponding to an estimation target item in the plurality of input rules different from each other corresponding to the plurality of items, thereby acquiring a converted face image corresponding to the estimation target item, and
   the information acquisition unit inputs the converted face image to the estimating model to acquire skin condition information of the subject regarding the estimation target item.
<15> The skin condition estimating system according to any one of <8> to <14>, further including a user terminal, wherein
   the user terminal includes at least the image acquisition unit, the skin condition output unit, and the information display unit.
<16> The skin condition estimating system according to any one of <11> to <13>, further including the user terminal, wherein
   the user terminal includes at least the image acquisition unit, the skin condition output unit, the information display unit, and the position identification unit.
<17> A user terminal for the skin condition estimating system according to any one of <8> to <14> includes at least the image acquisition unit, the skin condition output unit, and the information display unit.
<18> A user terminal for the skin condition estimating system according to any one of <11> to <13> includes the image acquisition unit, the skin condition output unit, the information display unit, and the position identification unit.
<19> The user terminal according to <17> or <18>, further including the conversion unit.

Hereinafter, the above-described contents will be described in more detail with reference to examples. However, the description of the following examples does not add any limitation to the contents described above.

### EXAMPLES

In the present example, 2207 face images (hereinafter, it is referred to as face image samples) obtained by shooting 353 Japanese women in a plurality of photographing environments using a plurality of kinds of photographing devices were prepared, and the accuracy of the skin condition estimating method (the present method) according to the present embodiment described above was verified using the face image samples. The plurality of types of photographing devices includes a single-lens reflex digital camera, a smartphone, and a tablet terminal.

The verification of the accuracy of the present method was performed by comparing it with the accuracy of an existing general method (hereinafter, it is referred to as a conventional method) for estimating the skin condition of the human face appearing in the face image and the accuracy of the method described in Patent Document 1 described above (described as the prior patent method). Specifically, an AI model was constructed and learned for each of the present method, the conventional method, and the prior patent method, and the accuracy of the skin condition estimated by each AI model was compared.

Each AI model was constructed by performing the fine-tuning of the VGG16 based on a learned model of a convolutional neural network (CNN) for ImageNet. The learning condition of each AI model was different only in the face image used as the training data, and the other conditions were the same. As a learning method, Cosine Annealing having 20 epochs as one cycle from a learning rate (lr) of 0.001 to 0.0001 was used, and the total number of learnings was set to 1000 epochs. In addition, a mean squared error was used for the Loss function, and a stochastic gradient descent (SGD) method was used as an optimization algorithm.

Each AI model was formed by four CNNs so that the skin conditions of 18 items can be estimated.

The estimation items of the skin condition are makeup feeling, skin age, male/female skin likeness, makeup smudge, powdery, glossy, wet, comprehensive evaluation, visual wetness, visual dryness, smoothness, fineness, luster, firmness, transparency, skin tone, skin yellowness, and dullness.

The estimation item output from one CNN was determined with reference to the collinearity between the estimation items and the learning efficiency in a case where learning is performed in combination. Specifically, an AI model was formed such that a combination of items, such as fineness, smoothness, and transparency, assumed to have high linkage or collinearity with each other, was output by one CNN, and a combination between items, for which estimation accuracy becomes high by performing learning of the AI model, was searched. As a result, an AI model in which four CNNs estimate skin conditions of 18 items was constructed.

The correct answer information of the training data of each AI model is acquired by a professional evaluator visually evaluating the skin condition of the face of the subject or applying a specific analysis method to the face image of the subject photographed by a dedicated skin image measuring apparatus.

Each original face image included in the face image sample was converted by each method of the present method, the conventional method, and the prior patent method. That is, a group of 2207 face images converted by the present method, a group of 2207 face images converted by the conventional method, and a group of 2207 face images converted by the prior patent method were prepared.

Each face image group prepared for each method was divided into 5: 3: 2 for learning, verification, and test, respectively, 50% of the face image group for learning was used as training data of the AI model, 30% of the face image group for verification was used for selection of the learned AI model, and estimation accuracy of each AI model was evaluated using 20% of the face image group for test. In addition, the face image group for learning is multiplied by 10 by performing random rotation processing of maximum ±5 degrees on each face image.

In each AI model, the image size of the input image is defined as 224 pixels (the above-described predetermined image size), and the shape of the input image is defined as a square (the above-described predetermined rectangular shape).

A face image conversion method used in each method is as follows.

In the conventional method, a face image (image size: 224 pixels, image shape: square) in which an original face image of a face image sample is normalized in terms of an image size and a shape and only alignment is performed such that a face comes to a predetermined position by face recognition is used. In the prior patent method, in addition to the normalization of the conventional technique and the alignment of the face by face recognition, processing of filling a region other than the face region (background or hair) and each region of the eyes, the eyebrows, and the lips in a skin color is performed on the original face image.

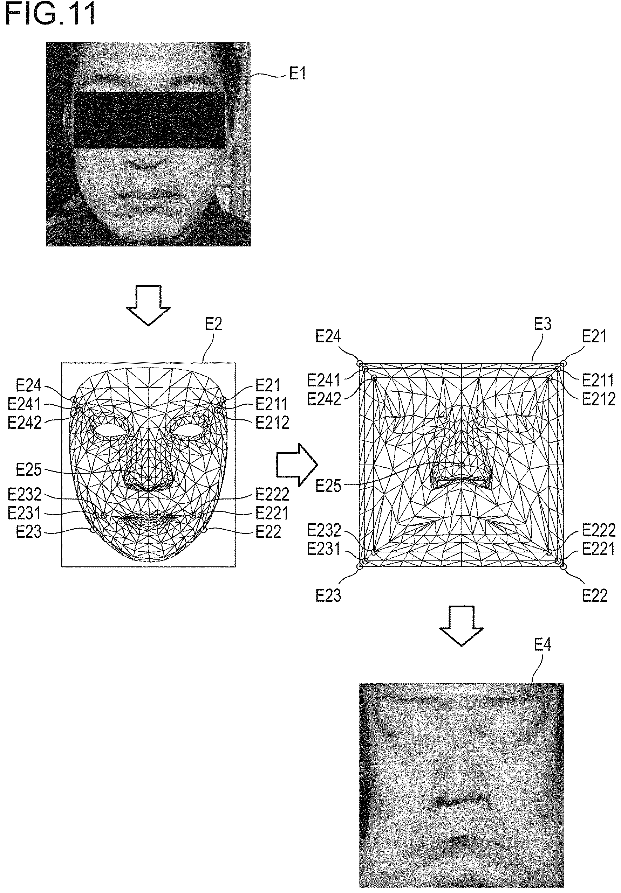

In the present example, the present method converted the face image as illustrated in Fig. 11. Fig. 11 is a diagram illustrating a flow of conversion of a face image in a skin condition estimating method according to the present example.

In the position identification process (S22), position coordinates of 468 landmarks in the face region in the original face image are identified using the Google (registered trademark) FaceMesh. In the example of Fig. 11, a mesh formed by connecting points of the respective landmarks with line segments is illustrated in reference numeral E2.

The detected landmark includes a point indicating the contour of the face, a vertex of the nose, a point indicating the contours of the eyes, the eyebrows, and the lips, and the like. The two points adjacent to the outer ends of the left and right eyebrows on the contour of the face are denoted by reference numerals E21 and E24, the two points adjacent to the left and right corners of the mouth on the contour of the face are denoted by reference numerals E22 and E23, and the nose vertex is denoted by reference numeral E25. The landmarks denoted by reference numerals E21 to E24 correspond to the above-described first contour points.

In addition, four points adjacent to the inside of the first contour points (E21 to E24) are denoted by reference numerals E211, E221, E231, and E241, which are referred to as second contour points.

Four points adjacent further inside the second contour points E211, E221, E231, and E241 are denoted by reference numerals E212, E222, E232, and E242, and are denoted as third contour points.

In the conversion process (S23), the position coordinates of the landmark identified in the process (S22) are coordinate-converted as indicated by reference numeral E3 in Fig. 11.

Specifically, the four first contour points E21, E22, E23, and E24 are respectively arranged at the vertices of the square in the converted face image, the nose vertex E25 is arranged at the center of the converted face image, and the other points on the contour of the face are arranged on the sides of the converted face image.

Furthermore, the four second contour points E211, E221, E231, and E241 and the four third contour points E212, E222, E232, and E242 are arranged on the diagonal lines connecting the four first contour points E21, E22, E23, and E24 and the nose vertex E25, respectively, and are arranged such that the difference between the distance from the nose vertex to the first contour point and the distance from the nose vertex to the second contour point is smaller than the difference between the distance from the nose vertex to the second contour point and the distance from the nose vertex to the third contour point.

The other landmarks are linearly moved in accordance with the movement of the point on the contour of the face region and the nose vertex while maintaining the mutual positional relationship as much as possible.

In the present example, by such coordinate conversion, the original face image is converted such that the face region occupies the entire square face image after the conversion, as indicated by reference numeral E4 in Fig. 11.

Furthermore, the original face image is converted by the coordinate conversion of the second and third contour points such that the closer to the contour of the face, the smaller the region, and the closer to the center of the face, the larger the region in the conversion face image.

As a result, since the face region is enlarged around the region where the presentation degree of the human face skin condition is high, it is possible to sufficiently suppress a decrease in the resolution of the region where the presentation degree of the skin condition is high and to enhance the estimation accuracy of the AI model even in a case where the image size is compressed in normalization of the face image.

In the example of Fig. 11, a cheek region among the face regions is enlarged more greatly while maintaining the mesh shape (on the triangle) as much as possible. This is because the presentation degree of the skin condition of the human face in the cheek region is high.

Furthermore, in the face image after conversion indicated by reference numeral E4 in Fig. 11, the reason why a black region that is not considered as a face region exists in the left, right, and upper edges of the square is that the position coordinates of the landmark specified in process (S22) are identified slightly outside the contour of the face region in the face image. As described above, the present invention can achieve the above-described effects while allowing a slight deviation regarding the identification of the position coordinates of the face region and the arrangement of the face region in the converted face image.

Furthermore, in the face image after the conversion, as indicated by reference numeral E4 in Fig. 11, the eye region, the eyebrow region, and the lip region are crushed. In the present example, among the landmarks on the contour of the lip region, each point on the upper contour of the upper lip region and each point on the lower contour of the lower lip region, which exist at positions vertically facing each other, are coordinate-converted to the same position coordinates, thereby the lip region of the converted face image is crushed. Furthermore, among the landmarks on the contour of the eyebrow region, each point on the upper contour of the eyebrow region and each point on the lower contour of the eyebrow region, which exist at positions facing each other vertically, are coordinate-converted to the same position coordinates, whereby the eyebrow region of the converted face image is crushed.

On the other hand, for the eye region, an outer peripheral landmark (hereinafter, it is referred to as an eye region outer peripheral point) surrounding the eye region on the outer side of points on the contour of the eye region is used. That is, by coordinate-converting the eye region outer peripheral point of the upper eyelid and the eye region outer peripheral point of the lower eyelid existing at positions vertically facing each other into the same position coordinates, the eye region of the converted face image is crushed.

In this manner, it is possible to enlarge the skin region in the face image while excluding the region, namely, the eyebrow region and the lip region, irrelevant to the skin condition of the human face from the input face image to the AI model and the face image of the training data. As a result, it is possible to prevent a decrease in the resolution of the face region at the time of input to the AI model, and eventually, it is possible to improve the estimation accuracy of the AI model.

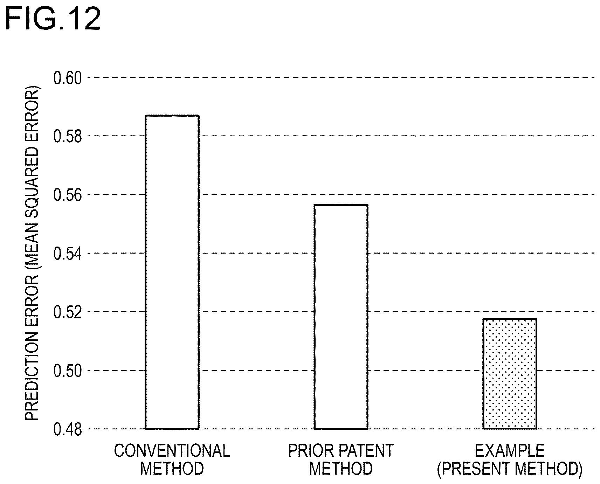

Fig. 12 is a graph illustrating a difference in prediction error among the skin condition estimating method (the present method) according to the present embodiment, the conventional method, and the prior patent method (WO 2018/222808 A) based on sensory evaluation by an expert. Fig. 12 illustrates a mean squared error.

For this comparison, a data set including a face image and impression evaluation items associated with the image was used. A total of 2207 face images including images under a plurality of photographing conditions such as an image shot in a predetermined photographing environment or images shot by the subject himself/herself in an arbitrary environment using his/her smartphone were used. For all of the images, an impression assessment score corresponding to each of the images was associated with each of the images using a visual assessment by a professional evaluator or a machine learning model and measurement device, the machine learning model pre-learned to predict a value of the visual assessment. For the three methods of the conventional method, the prior patent method, and the present method, the AI model was learned so that the prediction error of the impression evaluation item was minimized with respect to the sensory evaluation by the expert, and the mean squared error of the impression assessment score and the prediction value was calculated.

According to Fig. 12, the error of the present method is the smallest, the error of the prior patent method is the second smallest, and the error of the conventional method is the largest.

Table 1 indicates the results of comparing how much the mean squared error differs between the three methods of the conventional method, the prior patent method, and the present method with the mean squared error in the conventional method as 100% for the items of firmness, luster, skin surface smoothness, skin tone, skin yellowness, and dullness. Among them, the items of luster, skin surface smoothness, skin tone, and skin yellowness were preferable results.

**[Table 1]**

| MEAN SQUARED ERROR | FIRMNESS | LUSTER | SKIN SURFACE SMOOTHNESS | SKIN TONE | SKIN YELLOWNESS | DULLNESS |
|---|---|---|---|---|---|---|
| CONVENTIONAL METHOD | 100.00% | 100.00% | 100.00% | 100.00% | 100.00% | 100.00% |
| PRIOR PATENT METHOD | 88.20% | 101.70% | 98.20% | 102.40% | 89.90% | 88.10% |
| EXAMPLE (PRESENT METHOD) | 88.00% | 99.00% | 88.90% | 82.00% | 83.70% | 88.30% |

Therefore, according to the present example, it has been demonstrated that the estimation accuracy of the skin condition by the AI model can be improved by using the face image converted by the present method.

In addition, in the present example, a case where an original face image of the face image sample described above is converted using a coordinate conversion rule different from the coordinate conversion rule illustrated in Fig. 11 has also been examined.

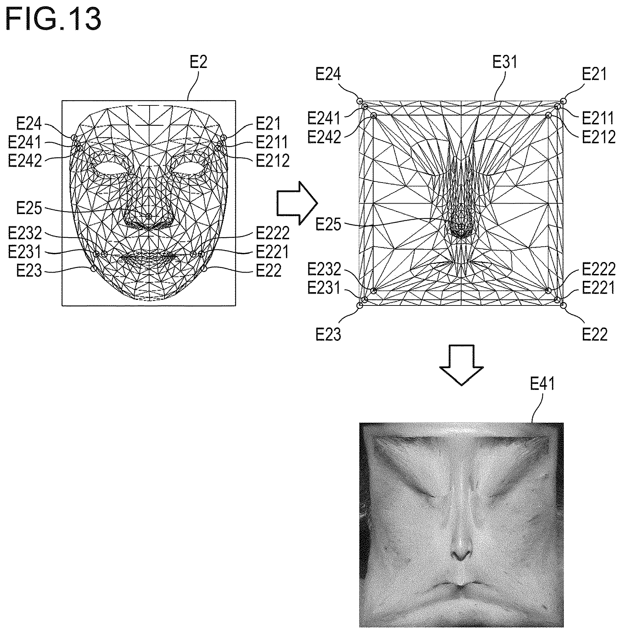

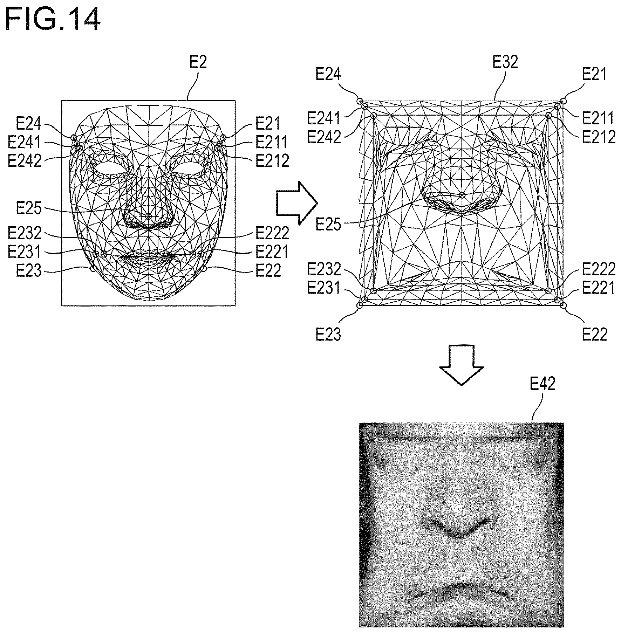

Fig. 13 is a diagram illustrating an example of face image conversion using the coordinate conversion rule that enlarges some meshes around the outer corner of the eye and immediately inside E212 and E242 more than other parts in order to estimate the wrinkle of the outer corner of the eye in more detail, in other words, that increases the enlargement ratio, and Fig. 14 is a diagram illustrating an example of face image conversion using a coordinate conversion rule that enlarges the part around the nasolabial fold, specifically, below E25 more than other parts in order to estimate the state of the nasolabial fold in more detail, in other words, that increases the enlargement ratio.

Even if the face images E41 and E42 converted using such coordinate conversion rules are used, the skin condition of the human face can be estimated similarly to the case of the above-described example.

Note that, in a case where the face images E41 and E42 are used, since the wrinkle region at the outer corner of the eye or the nasolabial region is enlarged, for example, the degree of conspicuousness of the nasolabial fold or the wrinkle at the inner corner of the eye can be more accurately evaluated as the skin condition presented in the region.

This application claims priority based on Japanese Patent Application No. 2022-124831 filed on August 4, 2022 and Japanese Patent Application No. 2023-52673 filed on March 29, 2023, the disclosures of which are all incorporated herein.

### REFERENCE SIGNS LIST

5 Communication line
7 AI model
10 Information processing apparatus (skin condition estimating apparatus) (skin condition estimating model learning device)
11 CPU
12 Memory
13 Input/output I/F
14 Communication unit
15 Display device
16 Input device
20 Server device
30 User terminal
31 Image acquisition unit (camera)
32 Position identification unit
33 Conversion unit
34 Information acquisition unit
35 Information display unit (display device)

## Claims

1. A skin condition estimating method using an estimating model learned to output a skin condition of a human face appearing in an input face image from the input face image, wherein
one or more processors execute:
an image acquisition process of acquiring a face image of a subject;
a conversion process of converting the face image so that a plurality of points on a contour of the face region of the human face appearing in the acquired face image is positioned at an outer edge of an image in order to match an input rule in the estimating model to obtain a converted face image; and
an information acquisition process of acquiring skin condition information of the face of the subject output from the estimating model by inputting the converted face image as the input face image.

2. The skin condition estimating method according to claim 1, wherein
in the converted face image, a predetermined local part region in a face region of a human face appearing in the acquired face image is removed or reduced, and
in the conversion process, positions of outer peripheral points facing each other via the predetermined local part region among a plurality of outer peripheral points surrounding the predetermined local part region in the face region are matched, and the predetermined local part region in the face region is removed or reduced.

3. The skin condition estimating method according to claim 2, wherein
the predetermined local part region includes an eye region, an eyebrow region, and a lip region.

4. The skin condition estimating method according to claim 2 or 3, wherein
the one or more processors further execute a position identification process of identifying position coordinates of a plurality of predetermined points in the face region of the human face appearing in the acquired face image, and
in the conversion process, the acquired face image is converted so as to conform to the input rule of the estimating model by coordinate-converting some or all of the identified position coordinates of the plurality of predetermined points.

5. The skin condition estimating method according to claim 4, wherein
the input face image has a predetermined rectangular shape,
the position coordinates of the plurality of predetermined points further include position coordinates of the plurality of points on the contour of the face region, the nose vertex of the face region, and a plurality of other points of the face region, and
in the conversion process, the coordinate conversion is performed such that each of four first contour points included in the plurality of points on the contour of the face region is arranged at a vertex of the predetermined rectangular shape, the nose vertex is arranged at a center of the predetermined rectangular shape, another point on the contour of the face region is arranged on a side of the predetermined rectangular shape, and the plurality of other points of the face region moves corresponding to the movement of the nose vertex and the plurality of points on the contour of the face region.

6. The skin condition estimating method according to claim 5, wherein
the plurality of other points of the face region includes four third contour points located closer to the contour of the face region than the predetermined local part region and four second contour points located further closer to the contour of the face region than the four third contour points, and
in the conversion process, the four second contour points and the four third contour points are respectively arranged on diagonal lines connecting the four first contour points and the nose vertex, and the coordinate conversion is performed such that a difference between a distance of the first contour point from the nose vertex and a distance of the second contour point from the nose vertex is smaller than a difference between a distance of the second contour point from the nose vertex and a distance of the third contour point from the nose vertex.

7. The skin condition estimating method according to any one of claims 1 to 6, wherein
the estimating model is capable of estimating a plurality of items of the skin condition,
a plurality of the input rules of the estimating model is provided,
in the conversion process, the acquired face image is converted so as to correspond to the input rule corresponding to an estimation target item in the plurality of input rules different from each other corresponding to the plurality of items, thereby acquiring a converted face image corresponding to the estimation target item, and
in the information acquisition process, the converted face image is input to the estimating model to acquire skin condition information of the subject regarding the estimation target item.

8. A skin condition estimating system includes:
an estimating model learned to output a skin condition of a human face appearing in an input face image from the input face image;
an image acquisition unit configured to acquire a face image of a subject;
a conversion unit configured to obtain a converted face image by converting the face image so that a plurality of points on a contour of a face region of the human face appearing in the acquired face image is positioned at an outer edge of an image in order to match an input rule in the estimating model;
an information acquisition unit configured to input the converted face image to the estimating model as the input face image and acquire skin condition information of the face of the subject output from the estimation model;
a skin condition output unit configured to generate image information for presenting the acquired skin condition information; and
an information display unit configured to display the image information.

9. The skin condition estimating system according to claim 8, wherein
in the converted face image, a predetermined local part region in a face region of a human face appearing in the acquired face image is removed or reduced, and
the conversion unit matches positions of outer peripheral points facing each other via the predetermined local part region among a plurality of outer peripheral points surrounding the predetermined local part region in the face region to remove or reduce the predetermined local part region in the face region.

10. The skin condition estimating system according to claim 9, wherein
the predetermined local part region includes an eye region, an eyebrow region, and a lip region.

11. The skin condition estimating system according to claim 9 or 10, further comprising a position identification unit configured to identify position coordinates of a plurality of predetermined points in the face region of the human face appearing in the acquired face image, wherein
the conversion unit coordinate-converts some or all of the position coordinates of the plurality of predetermined identified points to convert the acquired face image so as to match the input rule of the estimating model.

12. The skin condition estimating system according to claim 11, wherein
the input face image has a predetermined rectangular shape,
the position coordinates of the plurality of predetermined points further include position coordinates of the plurality of points on the contour of the face region, the nose vertex of the face region, and a plurality of other points of the face region, and
the conversion unit performs the coordinate conversion such that each of four first contour points included in the plurality of points on the contour of the face region is arranged at a vertex of the predetermined rectangular shape, the nose vertex is arranged at a center of the predetermined rectangular shape, another point on the contour of the face region is arranged on a side of the predetermined rectangular shape, and the plurality of other points of the face region moves corresponding to the movement of the nose vertex and the plurality of points on the contour of the face region.

13. The skin condition estimating system according to claim 12, wherein
the plurality of other points of the face region includes four third contour points located closer to the contour of the face region than the predetermined local part region and four second contour points located further closer to the contour of the face region than the four third contour points, and
the conversion unit performs the coordinate conversion such that the four second contour points and the four third contour points are respectively arranged on diagonal lines connecting the four first contour points and the nose vertex, and a difference between a distance of the first contour point from the nose vertex and a distance of the second contour point from the nose vertex is smaller than a difference between a distance of the second contour point from the nose vertex and a distance of the third contour point from the nose vertex.

14. The skin condition estimating system according to any one of claims 8 to 13, wherein
the estimating model is capable of estimating a plurality of items of the skin condition,
a plurality of the input rules of the estimation model is provided,
the conversion unit converts the acquired face image so as to correspond to the input rule corresponding to an estimation target item in the plurality of input rules different from each other corresponding to the plurality of items, thereby acquiring a converted face image corresponding to the estimation target item, and
the information acquisition unit inputs the converted face image to the estimating model to acquire skin condition information of the subject regarding the estimation target item.

15. The skin condition estimating system according to any one of claims 8 to 14, further comprising a user terminal, wherein
the user terminal includes at least the image acquisition unit, the skin condition output unit, and the information display unit.

16. The skin condition estimating system according to any one of claims 11 to 13, further comprising a user terminal, wherein
the user terminal includes at least the image acquisition unit, the skin condition output unit, the information display unit, and the position identification unit.

17. A user terminal for the skin condition estimating system according to any one of claims 8 to 14, comprising at least the image acquisition unit, the skin condition output unit, and the information display unit.

18. A user terminal for the skin condition estimating system according to any one of claims 11 to 13, comprising the image acquisition unit, the skin condition output unit, the information display unit, and the position identification unit.

19. The user terminal according to claim 17 or 18, further comprising the conversion unit.
